# EUROPEAN PATENT SPECIFICATION

(11) **EP 4 117 709 B1**
(45) Date of publication and mention of the grant of the patent: **25.12.2024**
(21) Application number: 21712206.8
(22) Date of filing: 12.03.2021
(51) Int. Cl.: A61K 38/21, A61P 17/04, A61P 37/00

(54) **COMPOSITIONS AND METHODS RELATING TO THE TREATMENT OF DISEASES**
ZUSAMMENSETZUNGEN UND METHODEN IN DER BEHANDLUNG VON KRANKHEITEN
COMPOSITIONS ET MÉTHODES SE RAPPORTANT AU TRAITEMENT DE MALADIES

(30) Priority: 12.03.2020 GB 202003595
(43) Date of publication of application: 18.01.2023
(73) Proprietor: ILC Therapeutics Ltd, Newhouse Lanarkshire ML1 5UH (GB)
(72) Inventor: MCKENZIE, Christopher, Lanarkshire ML1 5UH (GB); STIMSON, William, Lanarkshire ML1 5UH (GB)
(74) Representative: Murgitroyd & Company
(86) International application number: PCT/GB2021/050630
(87) International publication number: WO 2021/181115

(56) References cited:
- EP-A2- 3 116 530
- WO-A1-2019/229480
- WO-A1-98/33517

## Description

### Field of the invention

The present invention relates to compositions and methods for preventing or treating pruritus, prurigo, neutrophilic dermatoses and skin cancer. The invention extends to compositions for preventing or treating conditions where an exaggerated Th2 response plays a detrimental role. The invention further extends to compositions and the use of the compositions of the invention for the treatment and/or prophylaxis of pruritus, prurigo and neutrophilic dermatoses for Human and Veterinary therapies.

### Background to the Invention

An over-reactive Th2 response may underlie inflammatory conditions, in particular pruritic inflammatory skin diseases. It is currently believed that Th2 cells play a major role in host defence against pathogens and an exaggerated Th2 response may lead to diseases, such as pruritus, prurigo and skin cancer, in particular cutaneous T-cell lymphoma.

Pruritus is defined as an unpleasant sensation that provokes the desire to scratch. It is a characteristic feature of many skin diseases and an unusual sign of some systemic diseases. Certain systemic diseases have long been known to cause pruritus that ranges in intensity from a mild annoyance to an intractable, disabling condition. Generalized pruritus may be classified into the following categories on the basis of the underlying causative disease: renal pruritus, cholestatic pruritus, hematologic pruritus, endocrine pruritus, pruritus related to malignancy, and idiopathic generalized pruritus. Pruritus, or itch, is most commonly associated with a primary skin disorder such as xerosis, urticaria, arthropod assault, mastocytosis, dermatitis herpetiformis, or pemphigoid.

Prurigo is a term used to denote a group of skin disorders characterised by intensely pruritic, and difficult to treat, papules or nodules. The best known of these conditions is prurigo nodularis, which typically presents with itchy nodules affecting the extremities, and consists histologically of hyperkeratosis and acanthosis, with downward projections of the epidermis. A similar condition, papular prurigo (papular dermatitis; chronic prurigo of adults) consists of smaller lesions, and presents mainly in middle-aged women.

Pruritus can be a symptom of some inflammatory and autoimmune dermatoses including conditions such as autoimmune bullous diseases and connective tissue diseases. Autoimmune bullous diseases (AIBD) are a heterogeneous group of severe dermatoses characterized by the presence of autoantibodies against cutaneous adhesion molecules and include Bullous Pemphigoid, Pemphigus Group and Dermatitis Herpetiformis. Connective tissues diseases include Systemic Sclerosis, Morphea, Systemic Lupus Erythematosus, Dermatomyositis, Sjogren Syndrome and Vitiligo.

Cutaneous T-cell lymphoma (CTCL) is a rare type of non-Hodgkin lymphoma that affects the skin. It develops when T-cells (also called T-lymphocytes) become abnormal. There are different types of CTCL, the most common are called mycosis fungoides and Sézary syndrome.

It is known that different pathogens induce different Interferon-alpha (IFN-α) subtypes *in vitro* and that IFN-α subtypes have different anti-viral, anti-proliferative and immunomodulatory activities. Infection via a variety of routes has been shown to induce different subtype profiles. IFN-α subtypes bind to the same receptors, activate common signaling pathways and had been expected to have similar immunological functions. All IFN-α subtypes have anti-viral activities, by definition, although their absolute efficacy in this context may vary considerably. In addition, many other biological properties have been described, but with varying potencies, including immunomodulatory and anti-proliferative activities. The pleiotropic effects appear to be due to differential interaction with the receptor chains and signaling through different intracellular pathways to an array of effector molecules. The Type I IFN receptor consists of two chains, IFNR1 and IFNR2. There is a range of binding affinities for each of the 12 IFN-α subtypes with the different receptor chains. IFNα-14 has one of the highest affinities for both of the two interferon receptors, which is why it is so active compared to the other 11 subtypes.

IFN-α may have a key role in the regulation of Th2 responses. It has been shown that IFN-α treatment suppresses Th2 cell development through the suppression of IL4 and IL13 gene expression. IFN-α therefore is able to re-establish a Th2 population balance in diseases and infections that promote a Th2 cell imbalance. In recent years, it became evident that besides its anti-viral effects, several immunomodulatory functions are exerted by IFN-α. IFN-α can impact on dendritic cell differentiation and controls the expression of various pro-inflammatory cytokines such as IL8 or IL18 and induces several anti-inflammatory mediators such as IL1 receptor antagonist (IL1Ra), soluble TNF receptor p55, IL10 and IL18 binding protein. However, the mechanisms of actions of IFN-α, and in particular individual IFN-α subtypes, are still only partly understood.

There is a significant unmet need for an effective, safe, topical treatment that can be used to treat pruritus and prurigo.

### Summary of the Invention

The present inventors submit that it would be desirable to develop an improved immunotherapeutic approach for the treatment and/or prophylaxis of pruritus, prurigo and neutrophilic dermatoses. Since pruritus, results from over-reactivity of Th2 cells and a corresponding overproduction of certain cytokines, a medication that is able to modify and balance a misdirected Th2 response and overproduction of related cytokines would be beneficial in treating pruritus. Such a medication would further be suitable to treat diseases and conditions where an exaggerated Th2 response plays a role, as in pruritus and prurigo. Neutrophilic dermatoses are a heterogenous group of inflammatory skin disorders characterised by sterile, predominately neutrophilc infiltrate on histopathology. It can be considered to be an overarching term for primarily epidermal or dermal processes with variable evidence of primary or secondary vasculitic changes. They comprise Sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, adult Still disease, primarily bullous, epidermal, and vasculitic NDs. The inventors consider the IFN-α14, for example SEQ ID NO:1 or a variant or fragment thereof, HYBRID 1, for example SEQ ID NO:2 or a variant or fragment thereof or HYBRID 2, for example SEQ ID NO:3 or a variant or fragment thereof as described herein have utility in treating neutrophil dermatoses as they demonstrate anti-myeloid cell (Neutrophil) activities.

The inventors submits that there is a need to provide a topical treatment that can turn off the cytokines/chemokines in the keratinocyte layer that are chemotactic to neutrophils and basophils/mast cells that cause pruritus and inflammatory disorders in the skin.

The present invention relates to compositions and methods for preventing or treating conditions where an exaggerated Th2 response plays a detrimental role, such as pruritus, prurigo and neutrophilic dermatoses. The invention further extends to the use of the compositions of the invention in the treatment and/or prophylaxis of pruritus and prurigo. Suitably the inflammatory disorders of the skin of the present invention may be distinguished from inflammation associated with infectious and neoplastic processes
Following extensive experimentation, the inventors of the present invention has surprisingly determined that administering IFN-α14, for example SEQ ID NO:1 or a variant or fragment thereof, HYBRID 1, for example SEQ ID NO:2 or a variant or fragment thereof or HYBRID 2, for example SEQ ID NO:3 or a variant or fragment thereof as described herein results in the suppression or inhibition of various cytokines associated with the immune response in pruritus. The inventors unexpectedly determined that IFN-α14, HYBRID 1 or HYBRID 2 can interact directly to turn off the cytokines in the keratinocyte layer that are chemotactic to neutrophils and basophils/mast cells that cause pruritus and other inflammatory disorders in the skin. The inventors demonstrated that IFN-α14, HYBRID 1 or HYBRID 2 inhibits these chemokines even under the influence of TNF-α. Surprisingly, this effect is demonstrated when IFN-α14, HYBRID 1 or HYBRID 2 are administered topically. IFN-α14 is a large molecule of 17,000 Daltons and it was unexpected that this molecule would pass through the skin. What was more surprising was that the inventors unexpectedly found that the effect of IFN-α14, HYBRID 1 or HYBRID 2 on chemokines in keratinocytes was observed when provided topically. The inventors considers that the topical effects of IFN-α14, HYBRID 1 or HYBRID 2 are more selective and useful for pruritus and inflammatory disorders of the skin compared to the more indiscriminate effect when IFN-α14, HYBRID 1 or HYBRID 2 are provided to whole blood - both in the pleiotropic affect and the fact more tissues are brought into contact with the IFN-α14, HYBRID 1 or HYBRID 2.

The inventors have also established that the recombinant IFN-hybrid molecules known herein as HYBRID 1 (SEQ ID: 2) and HYBRID 2 (SEQ ID NO: 3) also have a high binding affinity to the interferon receptors, and will demonstrate an effect on chemokines involved with pruritus and inflammatory disorders of the skin, including neutrophilic dermatoses, in particular to turn off or inhibit chemokines in the keratinocyte layer that are chemotactic to neutrophils and basophils/mast cells that cause pruritus in the skin.

The present inventors have examined the ability of the synthetic alpha-interferons, Interferon-alpha-14 (SEQ ID NO:1), HYBRID 1 (SEQ ID NO:2) and HYBRID 2 (SEQ ID NO:3), to inhibit the production of interleukin-31 (IL31). IL31 is a cytokine that plays an important role in the induction of pruritus and, without wishing to be bound by theory, is considered to be important in inflammatory disorders of the skin such as neutrophilic dermatoses. The inventors very surprisingly determined that IFN-α14 (SEQ ID NO:1), HYBRID 1 (SEQ ID NO:2) and HYBRID 2 (SEQ ID NO:3) inhibit IL31. The inventors have examined the inhibition of IL31 in comparison with Interferon-alpha-2b (Roferon). Surprisingly Interferon-alpha14 (SEQ ID NO:1), HYBRID 1 (SEQ ID NO:2) and HYBRID 2 (SEQ ID NO:3) are active against IL31 production and supress or inhibit IL31 production while Interferon-alpha2b (Roferon) shows no inhibitory activity.

Interferon subtypes IFN-α10 and IFN-α14 and hybrids thereof are discussed in PCT Publication Number WO2014/037717 and PCT Publication Number WO2015/136287. In particular, IFN-α10-IFN-α14 hybrids are disclosed that contain sequences characteristic of the IFN-α10 and IFN-α14 subtype binding sites based on a consensus backbone sequence of all 12 alpha-interferons. Interferon subtypes IFN-α10 and IFN-α14 and hybrids thereof are also discussed in PCT Publication Number WO2017/046583. In particular, IFN-α10-IFN-α14 hybrids are disclosed that have high affinity binding sites derived from IFN-α10 and IFN-α14 subtypes that are not based on a consensus sequence of all 12 IFN-α subtypes. The hybrids derive the sequence characteristics of IFN-α10 and IFN-α14 subtypes without the sequence characteristics of the other 10 interferon-alpha subtypes.

Whilst not wishing to be bound by theory, the inventors believe that proteins comprising the amino acid sequence of IFN-α10 have greater affinity to interferon receptor 2 (IFNR2) and proteins comprising the amino acid sequence of IFN-α14 have greater affinity to interferon receptor 1 (IFNR1). Thus, substitution of a protein comprising an IFN-α10 amino acid sequence with amino acids of IFN-α14 which allow binding to interferon receptor 1 or substitution of a protein comprising an IFN-α14 amino acid sequence with amino acids of IFN-α10 which allow binding to interferon receptor 2 is considered to provide a IFN-α10 IFN-α14 hybrid protein which should have stronger binding affinity to both interferon receptors 1 and 2 than IFN-α10 or IFN-α14 alone. By including the primary interferon receptor binding sites of IFN-α10 and IFN-α14 is meant that the hybrid comprises amino acids selected from IFN-α10 and substituted into an IFN-α14 amino acid sequence to improve the ability of an IFN-α14 subtype to bind to an interferon receptor 2 and / or that the hybrid comprises amino acids selected from IFN-α14 and substituted into an IFN-α10 amino acid sequence to improve the ability of an IFN-α10 subtype to bind to an interferon receptor 1.

Suitably, several amino acid substitutions of protein comprising an IFN-α10 amino acid sequence with amino acids of IFN-α14 determined to be involved in binding to interferon receptor 1 may enhance the binding of the protein to interferon receptor 1. Suitably, an amino acid substitution of protein comprising an IFN-α14 amino acid sequence with amino acids of IFN-α10 determined to be involved in binding to interferon receptor 2 may enhance the binding of the protein to interferon receptor 2.

In embodiments the IFN-α10-IFN-α14 hybrid can substantially have the amino-acid sequence of IFN-α10, but be modified in a region between amino residues 80 to 150, or suitably between amino acid residues 84 to 144, or suitably amino acid residues 92 to 115 or suitably between amino acid residues 90 to 110, (utilizing the numbering of the IFN-α10 sequence) to provide the amino acids provided by the IFN-α14 sequence. It is considered the amino acid residues in these regions or parts of these regions provide for the binding of IFN-α14 to interferon receptor 1. In particular, the hybrid sequence may include at least one, at least two, at least three, at least 4, at least 5, at least 6, at least 7, at least 8, at least 9, at least 10 or at least 11 modifications of the IFN-α10 sequence to provide the corresponding residues of the IFN-α14 sequence or a conserved mutation thereof. In embodiments, eleven modifications are provided as indicated by the amino acids noted in bold.

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD
(HYBRID 2: SEQ ID NO: 3).

In embodiments, the IFN-α10-IFN-α14 hybrid sequence may include at least one mutation selected from amino acids at positions 94, 101, 102, 109 or 144, preferably at least two mutations selected from amino acids at positions 94, 101, 102, 109 or 144, more preferably at least three mutations selected from amino acids at positions 94, 101, 102, 109 or 144, more preferably at least four mutations selected from amino acids at positions 94, 101, 102, 109 or 144 or more preferably at least five mutations selected from amino acids at positions 94, 101, 102, 109 or 144. In alternative embodiments, IFN-α14 can be utilised as a backbone structure of the hybrid and the residues which differ between the IFN-α10 and IFN-α14 sequences at the N and C terminal regions of the sequences can be provided in the hybrid sequence as those present in the IFN-α10 sequence. Suitably at least 1, at least 2, at least 3, at least 4, at least 5, at least 6, at least 7, at least 8, at least 9, at least 10 or at least 11 substitutions of the IFN-α14 N-terminal sequence may be made to provide the hybrid sequence to provide residues from IFN-α10 at those amino acid positions wherein the amino acids are not shared/common between IFN-α10 and IFN-α14. Suitably, at least 1, at least 2, or 3 substitutions are provided at the IFN-α14 C terminal sequence to provide residues from IFN-α10 to the hybrid sequence at those amino acid positions which are not shared/common between IFN-α10 and IFN-α14. In embodiments at least 1, at least 2, at least 3, at least 4, at least 5, at least 6, at least 7, at least 8, at least 9, at least 10 or at least 11 substitutions from the N-terminal sequence and at least 1, at least 2, or 3 substitutions from the C-terminal sequence of the IFN-α14 are made to provide residues from IFN-α10 to the hybrid at those amino acid positions which have amino acids that are not shared/common between IFN-α10 and IFN-α14.

In embodiments, the IFN-α14 comprises or consists of an amino acid sequence SEQ ID NO: 1 or a functionally active fragment or variant thereof.

In embodiments, the hybrid comprises or consists of an amino acid sequence SEQ ID NO: 2 or a functionally active fragment or variant thereof.

In embodiments, the hybrid comprises or consists of an amino acid sequence SEQ ID NO: 3 or a functionally active fragment or variant thereof.

By functionally active is meant an IFN-α10-IFN- α14 hybrid polypeptide comprising the primary interferon binding sites of IFN-α10 and IFN-α14 wherein the administration of peptide to a subject or expression of peptide in a subject promotes suppression of a Th2 mediated immune response. Further, functional activity may be indicated by the ability of a hybrid peptide to suppress a Th2 mediated response.

A fragment can comprise at least 50, preferably 100 and more preferably 150 or greater contiguous amino acids from SEQ ID NO: 1, 2 or 3 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein.

By variant is meant an amino acid sequence which is at least 70% homologous to SEQ ID NO: 1, 2 or 3, more preferably at least 80% homologous to SEQ ID NO: 1, 2 or 3, more preferably at least 90% homologous to SEQ ID NO: 1, 2 or 3, even more preferably at least 95% homologous to SEQ ID NO: 1, 2 or 3, even more preferably at least 96% homologous to SEQ ID NO: 1, 2 or 3, even more preferably at least 97% homologous to SEQ ID NO: 1, 2 or 3 and most preferably at least 98% homology with SEQ ID NO: 1, 2 or 3. A variant encompasses a polypeptide sequence of SEQ ID NO: 1, 2 or 3 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Hybrids, and variants and fragments thereof may be generated using suitable molecular biology methods as known in the art.

The inventors also consider that there is some relevance for the use of IFN-α14 (SEQ ID NO:1), HYBRID 1 (SEQ ID NO:2) or HYBRID 2 (SEQ ID NO:3) as a topical treatment in relation to the sub-epidermis layers of the skin. Without wishing to be bound by theory it is considered a portion of the IFN-α14, HYBRID 1 or HYBRID 2 can pass through the skin to the lower dermis layer, where there are many leucocytes, especially Th2 that releases IL31. IL31 induces chemokines and other cytokines in pruritic conditions via its surface receptor. IL31 is a potent pruritogenic cytokine and its systemic and local administration induces scratching behaviour in rodents, dogs and monkeys.

The inventors have surprisingly determined that administration of IFN-α14, HYBRID 1 or HYBRID 2, in particular SEQ ID NO:1, 2 or 3 or a variant or fragment thereof, as a topical treatment results in a greater reduction or inhibition of IL31 in keratinocytes compared to previous topical medications. In addition, the inventors have determined that very low doses of IFN-α14, HYBRID 1 or HYBRID 2 for example up to 5×10³ IU/ml or 5×10⁴ IU/ml topical cream can be used.

The inventors also indicate that as IFN-α14, in particular SEQ ID NO:1 or a variant or fragment thereof, diffuses down into the lower dermis layer it can also turn off or inhibit TNF-α which also results in inhibition of chemokines such as IL17, e.g. IL17A, IL17B, IL17F and/or IL22.

This has led to the identification by the inventors of improved therapeutic compositions which have utility in the treatment and/or prophylaxis of pruritus, prurigo and diseases and conditions where an exaggerated Th2 response plays a role.

The invention is as claimed in claims 1 to 8.

Disclosed is a method for the treatment and/or prophylaxis of pruritus, prurigo, nuceleophilic dermatoses or skin cancer, said method comprising the step of:
(i) administering to a subject in need thereof a therapeutically effective amount of an interferon alpha subtype, wherein the interferon alpha subtype is IFN-α14, HYBRID 1, HYBRID 2 or a combination of IFN-α14 and HYBRID 1 or HYBRID 2.

In embodiments, the interferon alpha subtype IFN-α14 comprises or consists of an amino acid sequence SEQ ID NO:1.

In embodiments, the interferon alpha subtype HYBRID 1 comprises or consists of an amino acid sequence SEQ ID NO:2.

In embodiments, the interferon alpha subtype HYBRID 2 comprises or consists of an amino acid sequence SEQ ID NO:3.

Disclosed is where the method of administration is topical administration. Disclosed is where the method of administration is sub-lingual. Without wishing to be bound by theory in both methods of administration it is considered that a concentration of IFN-α14, HYBRID 1 and HYBIRD 2 would be provided such that systemic effects of interferon are not induced. Thus, the chemokine and interleukin effects can be achieved without causing (or only minimally causing) antivral or anti-proliferative effects.

It would be considered this administration is distinguished from systemic delivery of interferons in the art which have provided pharmacological doses. Such pharmacological doses would activate anti-viral/bacterial properties of such interferons (for example as would have been observed following administration of IFNalpha2c in the art) - causing side effects and abrogating the low concentration-associated immune regulation effects observed by the inventors following topical administration. Typically, topical doses may be 100 -1000 x less than systemic doses and allow control of immune response in the skin compartment only.

In embodiments, the therapeutically effective amount of the interferon alpha subtype IFN-α14, HYBRID 1 and HYBRID 2 is a low dose (up to 5×10⁴ IU units or 5×10³ IU units/ml). In embodiments, the therapeutically effective amount of the interferon alpha subtype IFN-α14, HYBRID 1 and HYBRID 2 is lower than current systemic treatments for pruritus or other pruritic conditions or inflammatory disorders, for example neutrophilic dermatoses.

In embodiments, the interferon alpha subtype IFN-α14, HYBRID 1 and HYBRID 2 is administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml, 1×10⁷IU/ml or 1×10⁸IU/ml.

The inventors have elucidated that interferon alpha subtypes cause a varied response both from each other, and dependent on dose (high dose leading to systemic - anti-viral and anti-proliferative effects) and low dose-chemokine and interleukin effects at a non-systemic level and that response can vary dependent of tissue.

In embodiments, the interferon alpha subtype IFN-α14, HYBRID 1 and HYBRID 2 is administered in a dose of 0.1mg to 1mg, 1mg to 3mg, 3mg to 5mg or 5mg to 10mg. For example, in human topical applications 5×10⁴ IU/ml cream or less may be used. In animals, e.g. dog, sublingual use may be 10⁴ IU/Kg, for example in 1ml PBS.

In embodiments, the interferon alpha subtype IFN-α14, HYBRID 1 and HYBRID 2 is topically administered once a day, twice a day, three times a day or four times a day. Typically for sublingual administration, the dose would be provided once a day.

In embodiments, the interferon alpha subtype IFN-α14, HYBRID 1 and HYBRID 2 interacts directly to turn off or inhibit the cytokines/chemokines in the keratinocyte layer. In embodiments, the interferon alpha subtype IFN-α14, HYBRID 1 and HYBRID 2 interacts directly to turn off the cytokines in the keratinocyte layer that are chemotactic to neutrophils and basophils/mast cells that cause pruritus in the skin. In embodiments, the interferon alpha subtype IFN-α14, HYBRID 1 and HYBRID 2 passes through the skin to the lower dermis layer where it effects chemokine production.

In certain embodiments, the subject can be suffering from a condition where suppression of a Th2-mediated immune response is desired. In certain embodiments, the subject can be suffering from pruritus. In certain embodiments, the subject can be suffering from prurigo. In certain embodiments the subject can be suffering from neutrophilic dermatoses.

In embodiments, the pruritus is renal pruritus, cholestatic pruritus, hematologic pruritus, endocrine pruritus, pruritus related to malignancy, and idiopathic generalized pruritus. In embodiments, the pruritus is most commonly associated with a primary skin disorder such as xerosis, urticaria, arthropod assault, mastocytosis, dermatitis herpetiformis, or pemphigoid.

In embodiments, the prurigo is prurigo nodularis. In embodiments, the prurigo is papular prurigo (papular dermatitis; chronic prurigo of adults).

In embodiments the neutrophilic dermatoses may be selected from comprise Sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, adult Still disease, primarily bullous, epidermal, and vasculitic NDs.

In certain embodiments, the pruritus is a symptom of inflammatory or autoimmune dermatoses. In embodiments, the inflammatory or autoimmune dermatoses where pruritus is a symptom is an autoimmune bullous disease (AIBD) or a connective tissue disease. In embodiments, the autoimmune bullous diseases is Bullous Pemphigoid, Pemphigus Group or Dermatitis Herpetiformis. In embodiments, the connective tissues disease is Systemic Sclerosis, Morphea, Systemic Lupus Erythematosus, Dermatomyositis, Sjogren Syndrome or Vitiligo.

Typically, the subject is a mammal, in particular a human. In embodiments the subject can be an animal, for example, but not limited to a companion animal such as a dog.

According to a second aspect of the present invention, there is provided an interferon alpha subtype, wherein the interferon alpha subtype is IFN-α14, HYBRID 1 or HYBRID 2 or a combination of IFN-α14 and HYBRID 1 or HYBRID 2 for use in the treatment and/or prophylaxis of pruritus, prurigo or neutrophilic dermatoseswherein the interferon alpha subtype IFN-α14 comprises or consists of an amino acid sequence SEQ ID NO:1; wherein the interferon alpha subtype HYBRID 1 comprises or consists of an amino acid sequence SEQ ID NO:2; and wherein the interferon alpha subtype HYBRID 2 comprises or consists of an amino acid sequence SEQ ID NO:3.

In certain embodiments, the interferon alpha subtype will be administered topically. In certain embodiments the interferon alpha subtype may be administered sub-lingually. This may be particularly advantageous for veterinary treatments.

In embodiments, the interferon alpha subtype is administered at a low dose as discussed herein; wherein the low dose is up to 5×10³ IU/ml or 5×10⁴ IU/m. In embodiments, the interferon alpha subtype is administered at a very low dose. In embodiments, the therapeutically effective amount of the interferon alpha subtype is lower than current systemic treatments for pruritus.

In embodiments, the interferon alpha subtype can be administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml, 1×10⁷IU/ml or 1×10⁸IU/ml.

In embodiments, the the interferon alpha subtype can be administered in a dose of 0.1mg to 1mg, 1mg to 3mg, 3mg to 5mg or 5mg to 10mg.

In embodiments, the the interferon alpha subtype can be administered once a day, twice a day, three times a day or four times a day. Suitably, in sublingual administration, a single dose may be provided each day.

In embodiments, the pruritus is renal pruritus, cholestatic pruritus, hematologic pruritus, endocrine pruritus, pruritus related to malignancy, and idiopathic generalized pruritus. In embodiments, the pruritus is most commonly associated with a primary skin disorder such as xerosis, urticaria, arthropod assault, mastocytosis, dermatitis herpetiformis, or pemphigoid.

In embodiments, the prurigo is prurigo nodularis. In embodiments, the prurigo is papular prurigo (papular dermatitis; chronic prurigo of adults).

In embodiments the neutrophilic dermatoses may be comprise Sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, adult Still disease, primarily bullous, epidermal, and vasculitic NDs. In embodiments the neutrophilic dermatoses may be comprise Sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, and adult Still disease.

In certain embodiments, the pruritus is a symptom of an inflammatory or autoimmune dermatoses. In embodiments, the inflammatory or autoimmune dermatoses where pruritus is a symptom is an autoimmune bullous disease (AIBD) or a connective tissue disease. In embodiments, the autoimmune bullous diseases is Bullous Pemphigoid, Pemphigus Group or Dermatitis Herpetiformis. In embodiments, the connective tissues disease is Systemic Sclerosis, Morphea, Systemic Lupus Erythematosus, Dermatomyositis, Sjogren Syndrome or Vitiligo.

Also disclosed there is provided use of an interferon alpha subtype, wherein the interferon-alpha subtype is IFN-α14, HYBRID 1, HYBIRD 2 or a combination of IFN-14 and HYBRID 1 or HYBIRD 2, in the preparation of a medicament for the treatment and/or prophylaxis of pruritus, prurigo, neutrophilic dermatoses, skin cancer or a condition where suppression of a Th2-mediated immune response is desired.

According to a further aspect of the present invention, there is provided a composition comprising an interferon alpha subtype, wherein the interferon alpha subtype is IFN-α14, HYBRID 1, HYBRID 2 or a combination of IFN-α14 and HYBRID 1 or HYBRID 2, for use in the treatment and/or prophylaxis of pruritus, prurigo or neutrophilic dermatoses; wherein the IFN-α14 comprises or consists of an amino acid sequence SEQ ID NO:1; wherein the interferon alpha subtype HYBRID 1 comprises or consists of an amino acid sequence SEQ ID NO:2 and wherein the interferon alpha subtype HYBRID 2 comprises or consists of an amino acid sequence SEQ ID NO:3.

Also disclosed there is provided a pharmaceutical composition comprising an interferon alpha subtype, wherein the interferon alpha subtype is IFN-α14, HYBRID 1, HYBRID 2 or a combination of IFN-α14 and HYBRID 1 or HYBRID 2, for use in the treatment and/or prophylaxis of pruritus, prurigo, neutrophilic dermatoses, skin cancer or a condition where suppression of a Th2-mediated immune response is desired.

Also disclosed there is provided an interferon alpha subtype, wherein the interferon subtype is IFN-α14, HYBRID 1, HYBRID 2 or a combination of IFN-α14 and HYBRID 1 or HYBRID 2, for use in modulating an immune response, suitably modulating an immune response in the skin but not systemically in a subject.

In embodiments of the aspects of the invention outlined above, the interferon alpha subtype IFN-α14 comprises or consists of an amino acid sequence SEQ ID NO:1.

In embodiments of the aspects of the invention outlined above, the interferon alpha subtype HYBRID 1 comprises or consists of an amino acid sequence SEQ ID NO:2.

In embodiments of the aspects of the invention outlined above, the interferon alpha subtype HYBRID 2 comprises or consists of an amino acid sequence SEQ ID NO:3.

In embodiments of the aspects of the invention outlined above, the composition or pharmaceutical composition is administered topically. In certain embodiments the interferon alpha subtype may be administered sub-lingually. This may be particularly advantageous for veterinary treatments.

In embodiments of the aspects of the invention outlined above, the interferon alpha subtype is administered at a low dose. In embodiments, the interferon alpha subtype is administered at a very low dose. In embodiments, the therapeutically effective amount of the interferon alpha subtype is lower than current systemic treatments for pruritus.

In embodiments of the aspects of the invention outlined above, the the interferon alpha subtype is administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml, 1×10⁷IU/ml or 1×10⁸IU/ml.

In embodiments of the aspects of the invention outlined above, the the interferon alpha subtype is administered in a dose of 0.1mg to 1mg, 1mg to 3mg, 3mg to 5mg or 5mg to 10mg.

In embodiments of the aspects of the invention outlined above, the the interferon alpha subtype is administered once a day, twice a day, three times a day or four times a day. Suitably, in sublingual administration, a single dose may be provided each day.

In embodiments, the pruritus is renal pruritus, cholestatic pruritus, hematologic pruritus, endocrine pruritus, pruritus related to malignancy, and idiopathic generalized pruritus. In embodiments, the pruritus is most commonly associated with a primary skin disorder such as xerosis, urticaria, arthropod assault, mastocytosis, dermatitis herpetiformis, or pemphigoid.

In embodiments, the prurigo is prurigo nodularis. In embodiments, the prurigo is papular prurigo (papular dermatitis; chronic prurigo of adults).

In embodiments the neutrophilic dermatoses may be comprise Sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, adult Still disease, primarily bullous, epidermal, and vasculitic NDs. In embodiments the neutrophilic dermatoses may be comprise Sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, and adult Still disease.

In certain embodiments, the pruritus is a symptom of an inflammatory or autoimmune dermatoses. In embodiments, the inflammatory or autoimmune dermatoses where pruritus is a symptom is an autoimmune bullous disease (AIBD) or a connective tissue disease. In embodiments, the autoimmune bullous diseases is Bullous Pemphigoid, Pemphigus Group or Dermatitis Herpetiformis. In embodiments, the connective tissues disease is Systemic Sclerosis, Morphea, Systemic Lupus Erythematosus, Dermatomyositis, Sjogren Syndrome or Vitiligo.

In certain embodiments of the aspects of the invention outlined above, the IFN-α subtype comprises, consists of or is IFN-α14 such as a fusion protein, or recombinant protein or the like and in particular which comprises or consists of the amino acid sequence SEQ ID NO:1 or a variant or fragment thereof. In embodiments the IFN-α14 can be glycosylated.

In certain embodiments of the aspects of the invention outlined above, the IFN-α subtype comprises, consists of or is HYBRID 1 such as a fusion protein, or recombinant protein or the like and in particular which comprises or consists of the amino acid sequence SEQ ID NO:2.

In certain embodiments of the aspects of the invention outlined above, the IFN-α subtype comprises, consists of or is HYBRID 2 such as a fusion protein, or recombinant protein or the like and in particular which comprises or consists of the amino acid sequence SEQ ID NO:3.

In a further aspect of the invention there is provided a recombinant polypeptide comprising or consisting of SEQ ID NO:1. The invention extends to nucleic acid sequences derived from the amino acid sequence SEQ ID NO:1.

In a further aspect of the invention there is provided a recombinant polypeptide comprising or consisting of SEQ ID NO:2. The invention extends to nucleic acid sequences derived from the amino acid sequence SEQ ID NO:2.

In a further aspect of the invention there is provided a recombinant polypeptide comprising or consisting of SEQ ID NO:3. The invention extends to nucleic acid sequences derived from the amino acid sequence SEQ ID NO:3.

### Detailed description of the invention

The inventors of the present invention have surprisingly determined that administering IFN-α14, for example SEQ ID NO:1 or a variant or fragment thereof, IFN-α14/IFN-α10 hybrids such as HYBRID 1, for example SEQ ID NO:2 or a variant or fragment thereof or HYBRID 2, for example SEQ ID NO:3 or a variant or fragment thereof, as described herein results in the suppression or inhibition of various cytokines associated with the immune response in pruritus. Surprisingly, this effect is enhanced when the IFN-α14, HYBRID 1 or HYBRID 2 are administered topically.
SEQ ID NO:1 is IFNα-14 and can be defined as follows: 
CNLSQTHSLNNRRTLMLMA QMRRISPFSCLKDRHDFEFP
QEEFDGNQFQKAQAISVLHE MMQQTFNLFSTKNSSAAWDE
TLLEKFYIELFQQMNDLEAC VIQEVGVEETPLMNEDSILA
VKKYFQRITLYLMEKKYSPC AWEVVRAEIMRSLSFSTNLQ KRLRRKD
SEQ ID NO:2 is HYBRID 1 and can be defined as follows: 
CDLPQTHSLGNRRALILLGQMGRISPFSCLKDRHDFRIPQEEFDGNQFQKAQAISVLHEM
MQQTFNLFSTENSSAAWEQTLLEKFSIELFQQMNDLEACVIQEVGVEETPLMNEDSILAV
RKYFQRITLYLIERKYSPCAWEVVRAEIMRSLSFSTNLQKRLRRKD
SEQ ID NO:3 is HYBRID 2 and can be defined as follows:

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD

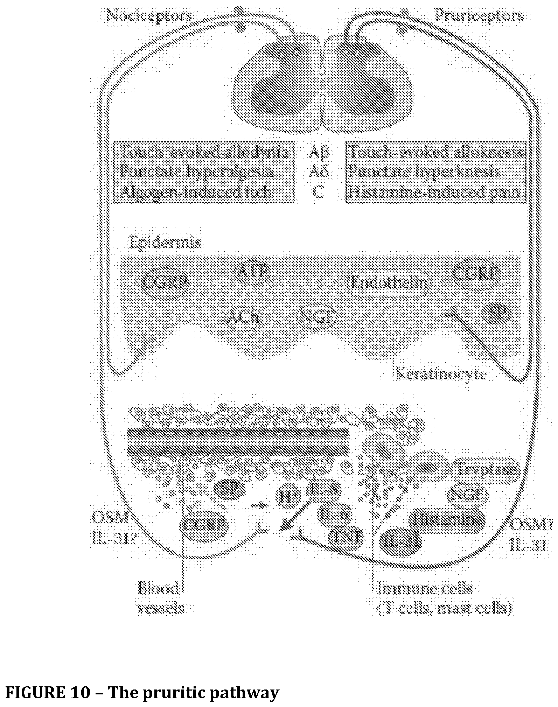

In particular, the inventors have determined that IFN-α14, in particular SEQ ID NO:1, HYBRID 1 (SEQ ID NO:2) or HYBRID 2 (SEQ ID NO:3) or a variant or fragment thereof, targets specific cytokines in keratinocytes associated with pruritus, for example IL31. IL31 is an inflammatory cytokine that helps trigger cell-mediated immunity against pathogens. IL31 is produced by a variety of cells, namely type 2 helper (Th2) T-cells. During Th2 dominated inflammation and in certain Th2 involved tumours (CTCL), as well as pruritus, prurigo, neutrophilic dermatoses and skin cancer, IL31 is released from activated skin homing Th2 cells. IL31 binds to the IL31RA receptor (IL31RA/OSMRβ receptor complex) on sensory nerve endings in the skin. As an amplification mechanism, IL31A communicates with ion channels such as TRPV1 that result in enhanced Ca signalling, STAT3 activation and probably other signal transduction pathways in sensory neurons. Figure 10 demonstrates the pruritic pathway.

The inventors have demonstrated that the natural molecule IFNα-14, in particular SEQ ID NO:1, SEQ ID NO:2, SEQ ID NO;3 or a variant or fragment thereof, eliminates or turns off IL31 in keratinocytes at very low doses. These findings can be applied to provide an improved method and improved composition for treating and/or preventing pruritus and prurigo.

In human keratinocytes, IL31 induces several chemokine genes that have been associated with atopic skin inflammation such as CCL1, CCL17 and CCL22. Hence, elevated levels of IL31 in pruritic lesions may enhance skin inflammation through the induction of chemokines, which subsequently lead to the recruitment of T cells. In turn, activated skin-infiltrating T cells may become new sources of IL31, thereby amplifying atopic skin inflammation and pruritus.

Pruritus or itchy skin is defined as an unpleasant sensation that provokes the desire to scratch. Generalized pruritus may be classified into the following categories on the basis of the underlying causative disease: renal pruritus, cholestatic pruritus, hematologic pruritus, endocrine pruritus, pruritus related to malignancy, and idiopathic generalized pruritus. Pruritus, or itch, is most commonly associated with a primary skin disorder such as xerosis, urticaria, arthropod assault, mastocytosis, dermatitis herpetiformis, or pemphigoid. Itch-induced scratching appears to exacerbate skin lesions in clinical and experimental dermatitis. Itching and scratching cause sleep loss and severely disturb the quality of life of affected individuals. Therefore, specifically treating itch or pruritus rather than simply treating the primary skin disorder is critically important in order to alleviate symptoms.

Itch is mediated by unmyelinated C-fibers and thinly myelinated Ad fibers originated from cell bodies in the dorsal root ganglion. Diverse endogenous and exogenous pruritogens such as histamine, 5-hydroxytryptamine, proteases, substance P, and chloroquine are known to induce the itch sensation. Each pruritogen mediates itch via specific receptor. Itch-specific peripheral neurons are positive for Mas-related G-protein-coupled receptor A3 (MargprA3) - this is known because genetic ablation of MargprA3-positive neurons attenuated scratch responses to chloroquine and histamine without affecting pain behaviors. The neuronal mechanism of itch sensation is not fully understood; however, the histaminergic itch pathway uses transient receptor potential cation channel vanilloid subtype 1 (Trpv1) as a direct downstream target. In non-histaminergic itch, some pruritogens (e.g. chloroquine) use transient receptor potential cation channel ankyrin subtype 1 (Trpa1).

Prurigo is a term used to denote a group of skin disorders characterised by intensely pruritic, and difficult to treat, papules or nodules. The best known of these conditions is prurigo nodularis, which typically presents with itchy nodules affecting the extremities, and consists histologically of hyperkeratosis and acanthosis, with downward projections of the epidermis. A similar condition, papular prurigo (papular dermatitis; chronic prurigo of adults) consists of smaller lesions, and presents mainly in middle-aged women.

Cutaneous T-cell lymphoma (CTCL) is a rare type of non-Hodgkin lymphoma that affects the skin. It develops when T-cells (also called T-lymphocytes) become abnormal. There are different types of CTCL, the most common are called mycosis fungoides and Sézary syndrome.

Inflammatory and autoimmune dermatoses where pruritus is a symptom include conditions such as autoimmune bullous diseases and connective tissue diseases. Autoimmune bullous diseases (AIBD) are a heterogeneous group of severe dermatoses characterized by the presence of autoantibodies against cutaneous adhesion molecules and include Bullous Pemphigoid, Pemphigus Group and Dermatitis Herpetiformis. Connective tissues diseases include Systemic Sclerosis, Morphea, Systemic Lupus Erythematosus, Dermatomyositis, Sjogren Syndrome and Vitiligo.

Besides chemical mediators, increasing research attention is being paid to interleukin-31 (IL31) and its receptor as an itch mediator since discovering the pruritogenic action of IL31 in mice in 2004. Indeed, systemic and local administration of IL31 induces scratching behavior in rodents, dogs, and cynomolgus monkeys. Prick testing with an IL31 solution triggers the itch sensation in humans.

Interleukin-4 (IL4) induces the gene expression and release of IL31 but details of all the cell types involved in IL31 production has remained unclear. Human dendritic cells are able to produce IL31 but to a much lesser extent than Th2 cells. Human basophils and mast cells also secrete IL31 and may cause chronic urticaria. Human eosinophils can also release IL31.

IL31 and its receptor, IL31RA, are highly expressed in various human and mouse cancer cell lines, as well as in tumour specimens from cancer patients. The IL31/IL31R axis has been shown to be involved in skin cancer, in particular cutaneous T cell lymphoma. Tumour cells were shown to produce IL31, whose serum levels correlated with pruritus intensity. Epidermal IL31 levels correlate to itch severity in cutaneous T-cell lymphoma.

The inventors consider that IFN-α14, HYBRID 1 and HYBRID 2 act on CXCL8 (IL8). IL8 a chemokine from keratinocytes that is an attractant for neutrophils, basophils and mast cells causing release of many tissue damaging substances. CXCL8 (IL8) is the primary cytokine involved in the recruitment of neutrophils to the site of damage or infection; a process called chemotaxis. A number of variables are essential for the successful chemotaxis of neutrophils, including the increased expression of high affinity adhesion molecules to secure the neutrophil to the endothelium near the affected site (and is, therefore, not washed away into the circulatory system), and that the neutrophil can digest its way through the basement membrane and the extracellular matrix (ECM) to reach affected site. CXCL8 plays a key role in inducing the cell signalling necessary to bring about these changes. IL8 is responsible for Histamine release from mast cells and basophils. At the site of infection histamine release causes vasodilation of the capillaries near the injured area which slows down the blood flow in the region and encourages leukocytes, such as neutrophils, to come closer to the endothelium, and away from the centre of the lumen where the rate of blood flow is highest. Once this occurs weak interactions are made between the selectins expressed on the neutrophil and endothelial cells (expression of which is also increased through the action of CXCL8 and other cytokines).

The inventors consider that IFN-α14, HYBRID 1 and HYBRID 2 act on IL6. IL6 is a growth factor from keratinocytes commonly associated with stress and fever. IL6 is an acute phase reactant and can be both pro- and anti-inflammatory. IL6 can act before IL17 production by inhibiting Th17 cell generation and after by suppressing the production of IL6 which increases keratinocyte proliferation.

The inventors have determined that administration of IFN-α14, in particular SEQ ID NO:1, HYBRID 1 (SEQ ID NO:2) or HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of IL31 compared to previous topical medications.

The inventors have surprisingly determined that administration of IFN-α14, in particular SEQ ID NO:1 or a variant or fragment thereof, results in the suppression of IL31, CXCL8 (IL8) or IL6 in keratinocytes by 50%, preferably by 60%, preferably by 70%, preferably by 80%, preferably by 90%, preferably by 91%, preferably by 92%, preferably by 93%, preferably by 94%, preferably by 95%, preferably by 96%, preferably by 97%, and more preferably by 98%.

The inventors have surprisingly determined that administration of IFN-α14, in particular SEQ ID NO:1, results in the suppression of IL31, CXCL8 (IL8) or IL6 keratinocytes at low doses as discussed herein. HYBRID 1 and HYBRID 2 show similar functional effects.

Treatment of the present invention can be administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml, 1×10⁷IU/ml or 1×10⁸IU/ml.

The treatment of the present invention can be administered in a dose of 0.1mg to 1mg, 1mg to 3mg, 3mg to 5mg or 5mg to 10mg.

The treatment of the present invention can be topically administered once a day, twice a day, three times a day or four times a day.

In addition, the inventors have determined that the administration or use of IFN-α14, in particular SEQ ID NO:1, HYBRID 1 (SEQ ID NO:2) or HYBRID 2 (SEQ ID NO:3), results in the full or partial inhibition of IL31 and/or the full or partial inhibition of CXCL8 (IL8) and/or the full or partial inhibition of IL6 in keratinocytes.

Moreover, the inventors have surprisingly determined that the topical administration of IFN-α14, HYBRID 1 or HYBRID 2, in particular SEQ ID NO:1, SEQ ID NO:2 or SEQ ID NO:3, can result in the targeting of the relevant cytokines in the keratinocyte layer as discussed herein. The present invention provides a superior topical treatment that is safe and effective in mild, moderate and severe pruritus. This treatment demonstrates a low side-effect profile. Low doses of the medication are required and the natural product, IFN-α14, HYBRID 1, or HYBRID 2 displays no cytotoxicity in-vitro at even the highest concentration (1×10⁸ IU/ml).

The inventors have demonstrated on keratinocytes from normal human skin cultures that were activated with TNF-α to induce chemokine (IL8) secretion, that IFNα-14, in particular SEQ ID NO:1, suppressed IL8 secretion directly by >80%. In addition, the inventors demonstrated that the addition of HYBRID 1, in particular SEQ ID NO:2 or HYBRID 2, in particular SEQ ID NO:3, , resulted in strong inhibition of the secretion of IL31. These results are a clear indication of the potential superiority of IFNα-14, in particular SEQ ID NO:1, HYBRID 1, in particular SEQ ID NO:2 or HYBRID 2, in particular SEQ ID NO:3, over existing systemic biologics.

The inventor, whilst not wishing to be bound by theory, has identified that the topical administration of IFN-α14, in particular SEQ ID NO:1, HYBRID 1, in particular SEQ ID NO:2 or HYBRID 2, in particular SEQ ID NO:3, can be used to treat pruritus and prurigo. The inventors have demonstrated that in spite of its relatively high molecular weight, IFNα-14, HYBRID 1 or HYBRID 2, in particular SEQ ID NO:1, SEQ ID NO:2 or SEQ ID NO:3, displays good permeation potential through the skin and, therefore, the development of clinically viable formulations that enable delivery of therapeutics doses of this peptide across the skin provides an unexpected approach to treat or prevent these pruritic conditions.

### Definitions

### Fragment

A fragment can comprise at least 50, preferably 100 and more preferably 150 or greater contiguous amino acids from SEQ ID NO: 1, SEQ ID NO:2 or SEQ ID NO:3 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein. Fragments may be generated using suitable molecular biology methods as known in the art.

### Variant

By variant is meant an amino acid sequence which is at least 70% homologous to SEQ ID NO: 1, SEQ ID NO:2 or SEQ ID NO:3, more preferably at least 80% homologous to SEQ ID NO: 1, SEQ ID NO:2 or SEQ ID NO:3, more preferably at least 90% homologous to SEQ ID NO: 1, SEQ ID NO:2 or SEQ ID NO:3, even more preferably at least 95% homologous to SEQ ID NO: 1, SEQ ID NO:2 or SEQ ID NO:3, even more preferably at least 96% homologous to SEQ ID NO: 1, SEQ ID NO:2 or SEQ ID NO:3, even more preferably at least 97% homologous to SEQ ID NO: 1, SEQ ID NO:2 or SEQ ID NO:3, and most preferably at least 98% homology with SEQ ID NO: 1, SEQ ID NO:2 or SEQ ID NO:3. A variant encompasses a polypeptide sequence of SEQ ID NO: 1, SEQ ID NO:2 or SEQ ID NO:3 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Variants may be generated using suitable molecular biology methods as known in the art.

### Subject

As herein defined, a "subject" includes and encompasses mammals such as humans, primates and livestock animals (e.g. sheep, pigs, cattle, horses, donkeys); laboratory test animals such as mice, rabbits, rats and guinea pigs; and companion animals such as dogs and cats.

### Treatment / Therapy

The term "treatment" is used herein to refer to any regimen that can benefit a human or non-human animal. The treatment may be in respect of pruritus, prurigo or skin cancer and the treatment may be prophylactic (preventative treatment). Treatment may include curative or alleviative effects. Reference herein to "therapeutic" and "prophylactic" treatment is to be considered in its broadest context. The term "therapeutic" does not necessarily imply that a subject is treated until total recovery. Similarly, "prophylactic" does not necessarily mean that the subject will not eventually contract a disease condition. Accordingly, therapeutic and/or prophylactic treatment includes amelioration of the symptoms of a particular pruritic condition or preventing or otherwise reducing the risk of developing a particular pruritic condition. The term "prophylactic" may be considered as reducing the severity or the onset of a particular condition. "Therapeutic" may also reduce the severity of an existing condition.

### Administration

The active ingredients used in the present invention in particular the interferon subtype IFN-α14, for example SEQ ID NO: 1, HYBRID 1 (SEQ ID NO:2) or HYBRID 2 (SEQ ID NO:3), as described herein can be administered separately to the same subject, optionally sequentially, or can be co-administered simultaneously as a pharmaceutical or immunogenic composition. The pharmaceutical composition will generally comprise a suitable pharmaceutical excipient, diluent or carrier selected depending on the intended route of administration.

The active ingredients can be administered to a patient in need of treatment via any suitable route. The precise dose will depend upon a number of factors, as is discussed below in more detail.

One suitable route of administration is topically, e.g. applied directly to the skin.

One suitable route is sublingual.

### Pharmaceutical Compositions

As described above, the present invention extends to a pharmaceutical composition for the treatment or pruritus, prurigo or skin cancer.

Pharmaceutical compositions according to the present invention, and for use in accordance with the present invention, may comprise, in addition to an active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. The formulation may be a liquid, for example, a physiologic salt solution containing non-phosphate buffer at pH 6.8-7.6, or a lyophilised or freeze-dried powder.

### Dose

The composition is preferably administered to an individual in a "therapeutically effective amount" or a "desired amount", this being sufficient to show benefit to the individual. As defined herein, the term an "effective amount" means an amount necessary to at least partly obtain the desired response, or to delay the onset or inhibit progression or halt altogether the onset or progression of a particular condition being treated. The amount varies depending upon the health and physical condition of the subject being treated, the taxonomic group of the subject being treated, the degree of protection desired, the formulation of the composition, the assessment of the medical situation and other relevant factors. It is expected that the amount will fall in a relatively broad range, which may be determined through routine trials. Prescription of treatment, e.g. decisions on dosage etc., is ultimately within the responsibility and at the discretion of general practitioners, physicians or other medical doctors, and typically takes account of the disorder to be treated, the condition of the individual patient, the site of delivery, the method of administration and other factors known to practitioners. The optimal dose can be determined by physicians based on a number of parameters including, for example, age, sex, weight, severity of the condition being treated, the active ingredient being administered and the route of administration. A broad range of doses may be applicable. Considering oral administration to a human patient, for example, from about 10 µg to about 1000 µg of agent may be administered per human dose, optionally for 3 to 4 doses. Dosage regimes may be adjusted to provide the optimum therapeutic response and reduce side effects. For example, several divided doses may be administered daily, weekly, monthly or other suitable time intervals or the dose may be proportionally reduced as indicated by the exigencies of the situation.

Unless otherwise defined, all technical and scientific terms used herein have the meaning commonly understood by a person who is skilled in the art in the field of the present invention.

### Autoimmune Disease

The term "autoimmune disease" as used herein is understood to mean any disease or condition which is caused by a body's tissues being attacked by its own immune system.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

The present invention will now be exemplified with reference to the following nonlimiting figures and examples which are provided for the purpose of illustration and are not intended to be construed as being limiting on the present invention. Other embodiments of this invention will be apparent to those of ordinary skill in the art in view of this description.

### Brief description of the Figures

Figure 1 shows a graph demonstrating the effect of IFNα-14 on CXCL8 (IL8) production from human keratinocytes.
Figure 2 shows a graph demonstrating the effect of IFNα-14 on IL6 production from human keratinocytes.
Figure 3 shows a graph demonstrating the effect of IFNα-14 on IL31 production from human leucocytes.
Figure 4 shows a graph demonstrating the effect of HYBRID 1 on IL31 production from human leucocytes.
Figure 5 shows a graph demonstrating the effect of HYBRID 2 on IL31 production from human leucocytes.
Figure 6 shows a graph demonstrating the effect of Human Alpha-Interferon-2a (Roferon) on IL31 production from human leucocytes.

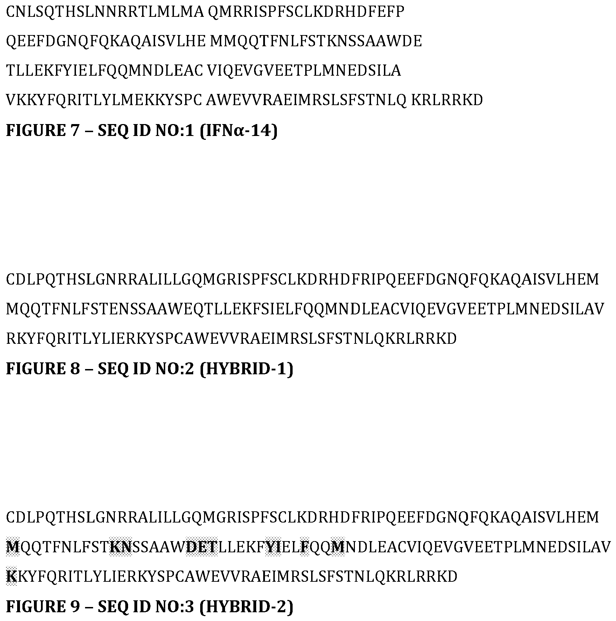

Figure 7 shows the IFN-α14 amino acid sequence.
Figure 8 shows the HYBRID 1 amino acid sequence.
Figure 9 shows the HYBRID 2 amino acid sequence.
Figure 10 demonstrates the pruritic pathway.

### Experimental Data

### Experiment 1: The effect of IFNα-14 on IL-6 and CXCL8 (IL8) production in ketatinocytes from normal human skin

The inventors tested the effect of IFNα-14 on keratinocytes from normal human skin that were activated with TNF-α to induce chemokine secretion.

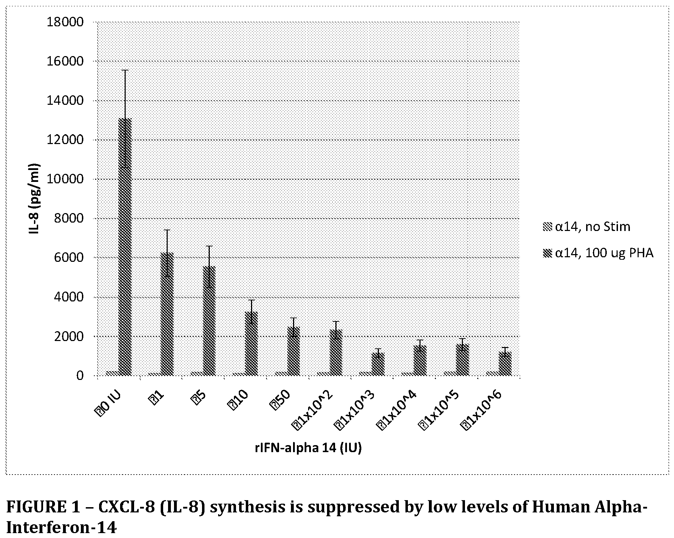

Figure 1 demonstrates that IFNα-14 suppresses CXCl8 (IL8) secretion directly by >80%. Figure 1 indicates strong inhibition of CXCL8 (IL8) in the presence of IFNα-14 and in particular at low concentrations of IFNα-14. CXCL8 is a major contributor to inflammation. It attracts neutrophils and basophils/mast cells to the site of inflammation - the latter release histamine and many other noxious agents e.g. prostaglandins, leukotrienes.

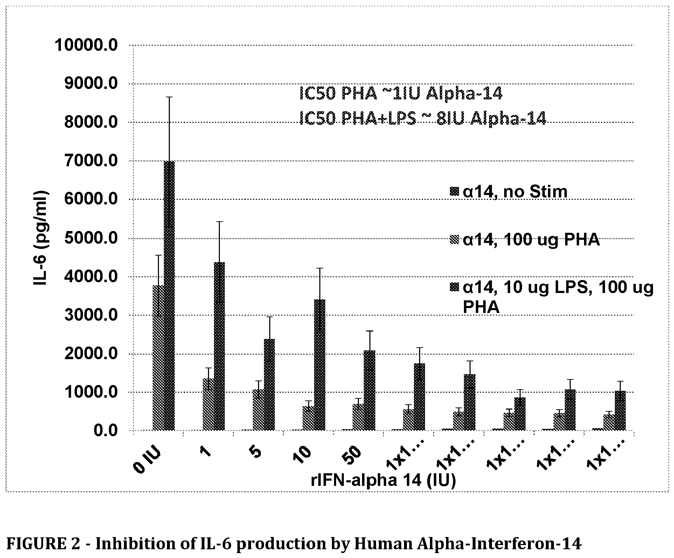

Figure 2 demonstrates inhibition of IL6 production in the presence of IFNα-14 and in particular at low concentrations of IFNα-14. IL-6 is an acute phase reactant that rises in trauma and is widely employed as an ancillary growth factor/stimulant. IL6 is a growth factor commonly associated with stress.

### Experiment 2: The effect of IFNα-14, HYBRID 1 and HYBRID2 on IL31 production in human leucocytes

### Whole blood Stimulation Assay

Fresh whole human blood (50IU/ml preservative heparin) was diluted 1/10 with RPMI 1640 medium with penicillin/streptomycin, L-glutamine and calcium. This was incubated with or without PHA-P (100 µg/ml) in the presence of a range of concentrations of either rIFN-alpha14, HYBRID 1 or HYBRID 2 for 24 hours at 37 °C in an atmosphere of 5% CO₂ in air in a humidified incubator. IFN concentrations used were 0, 1, 5, 10, 50, 100, 1,000, 10,000, 100,000 and 1,000,000 IU/ml. Each IFN concentration (1ml) was cultured in triplicate in 12 well plates.

After 24 hours incubation, the supernatants were harvested into individual labelled microcentrifuge tubes and centrifuged at 11,000 RPMI for 5 minutes in a microcentrifuge. Samples are then collected and stored in labelled microcentrifuge tubes at -20⁰C for assaying by ELISA.

### Typical ELISA procedure

One day prior to the beginning of the assay, a 96 well flat-bottomed ELISA plate was coated with 100 µl of the corresponding Capture Antibody, as per the manufacturer's instructions.

The following day, ELISA reagents and supernatants were allowed to heat to room temperature, and the coated ELISA plate was washed four times with wash buffer (1× phosphate buffer saline + 0.05%(v/v) Tween 20, and 200 µl assay diluent is added to each well to prevent nonspecific antibody binding, and incubated at room temperature for 1 hour, with shaking (500 rpm).

The plate was washed 4 times, and 100 µl of diluted standards and samples are added the appropriate wells. The plate is then sealed and incubated for 2 hours with shaking.

After a further 4 wash cycles, 100 ul of Detection Antibody was added to each well, followed by a further 1 hour incubation at room temperature, with shaking. The plate was washed a further 4 times, and diluted Avidin-HRP solution was added to each well, followed by a 30 minute incubation at room temperature, with shaking. The plate was then washed a total of 5 times, allowing for 30 seconds to 1 minute of soaking between washes, before adding 100 µl Substrate Solution for 15 minutes in darkness.

After 15-45 minutes, or when the standard wells reach the desired colour, 100 µl of stop solution (1M H₂SO₄) and the plate was read via spectrophotometry, typically at wavelengths 450 and 570 nm.

### Results

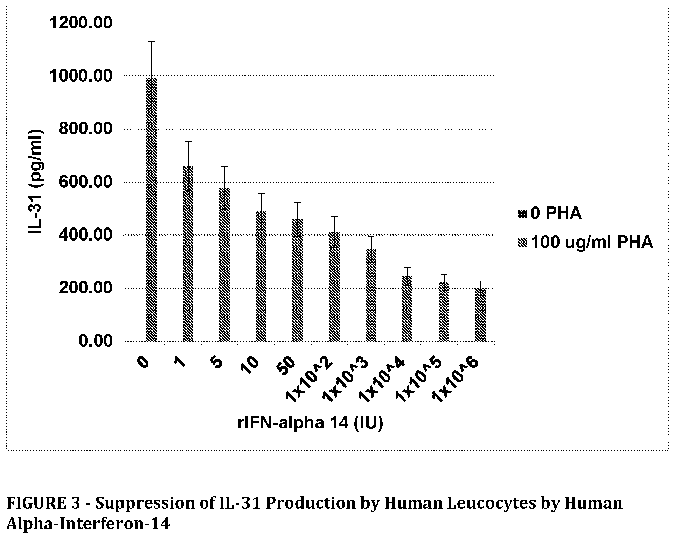

The results obtained are shown in Figures 3 to 5. Figure 3 demonstrates that IFNα-14 inhibits IL31 production in human leucocytes. Figure 3 indicates strong inhibition of IL31 in the presence of IFNα-14 and in particular at low concentrations of IFNα-14.

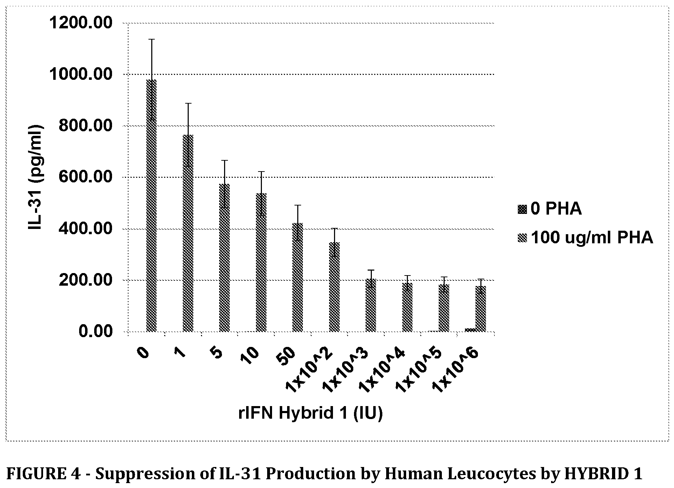

Figure 4 demonstrates that HYBRID 1 inhibits IL31 production in human leucocytes. Figure 4 indicates strong inhibition of IL31 in the presence of HYBRID 1 and in particular at low concentrations of HYBRID 1.

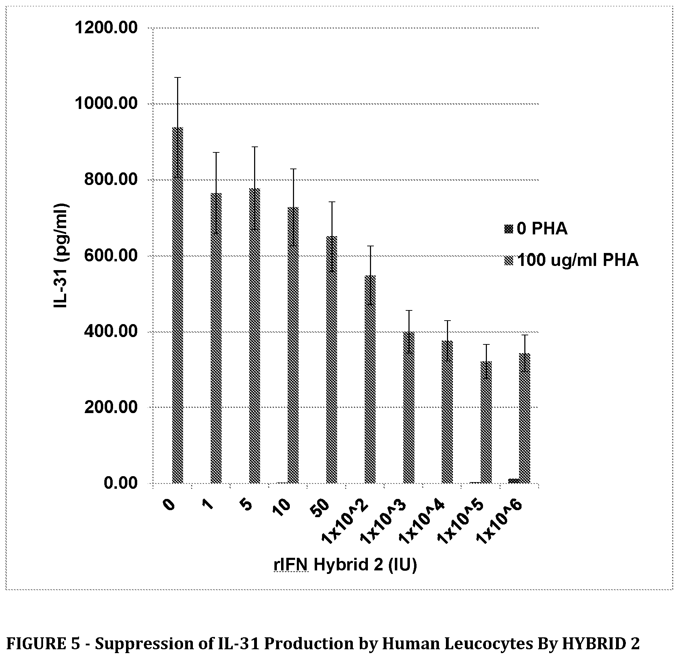

Figure 5 demonstrates that HYBRID 2 inhibits IL31 production in human leucocytes. Figure 5 indicates strong inhibition of IL31 in the presence of HYBRID 2 and in particular at low concentrations of HYBRID 2.

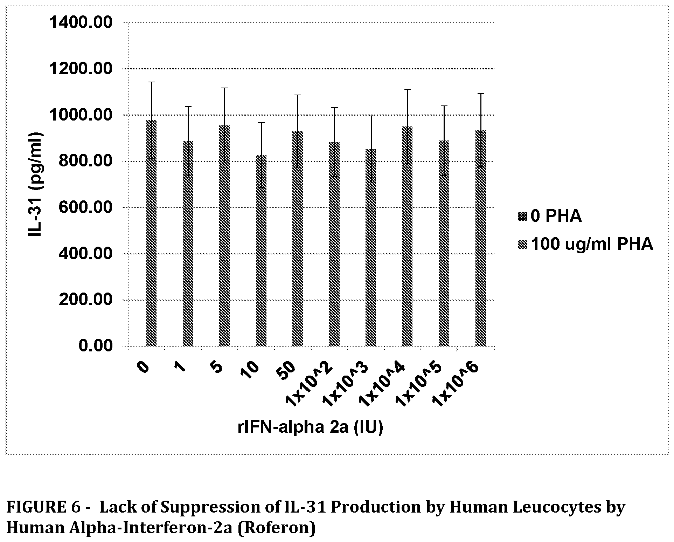

By way of comparison, the inventors used Human Alpha-Interferon-2a (Roferon) to demonstrate that it would be unexpected for an interferon alpha subtype to inhibit IL31. Figure 6 demonstrates a lack of suppression of IL-31 production by Human Alpha-Interferon-2a (Roferon) in human leukocytes.

These results surprisingly demonstrate that administration of IFN-α14, HYBRID 1 or HYBRID 2 results in a greater reduction or inhibition of IL31 in keratinocytes compared to previous medications.

## Claims

1. An interferon alpha subtype, wherein the interferon alpha subtype is IFN-α14, HYBRID 1, HYBRID 2 or a combination of at least two of IFN-α14, HYBRID 1, and HYBRID 2 for use in the treatment and/or prophylaxis of pruritus, prurigo or neutrophilic dermatoses; wherein the IFN-α14 comprises or consists of an amino acid sequence SEQ ID NO:1; wherein the interferon alpha subtype HYBRID 1 comprises or consists of an amino acid sequence SEQ ID NO:2; and wherein the interferon alpha subtype HYBRID 2 comprises or consists of an amino acid sequence SEQ ID NO:3.

2. The interferon alpha subtype of claim 1 for use in the treatment and/or prophylaxis of pruritus, prurigo or neutrophilic dermatoses, wherein the interferon alpha subtype is administered topically or is by sublingual administration.

3. The interferon alpha subtype of claim 2 for use in the treatment and/or prophylaxis of pruritus, prurigo or neutrophilic dermatoses, wherein the interferon alpha subtype is administered at a low dose; wherein the low dose is up to 5×10³ IU/ml or 5×10⁴ IU/ml.

4. The interferon alpha subtype of any of claims 1 to 3 for use in the treatment of a condition selected from pruritus, or prurigo.

5. The interferon alpha subtype of any of claims 1 to 3 for use in the treatment of neutrophilic dermatoses.

6. The interferon alpha subtype of claim 5 for use in the treatment of neutrophilic dermatoses wherein the neutrophilic dermatoses are selected from the list comprising sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, adult Still disease, primarily bullous, epidermal, and vasculitic NDs.

7. The interferon alpha subtype of claim 5 for use in the treatment of neutrophilic dermatoses wherein the neutrophilic dermatoses are selected from the list comprising sweet syndrome (SS), neutrophilic eccrine hidradenitis (NEH), Behcet disease (BD), pyoderma gangrenosum (PG), bowel-associated dermatosis-arthritis syndrome, rheumatoid neutrophilic dermatitis, and adult Still disease.

8. A composition comprising an interferon alpha subtype, wherein the interferon alpha subtype is IFN-α14, HYBRID 1, HYBRID 2 or a combination of IFN-α14 and HYBRID 1 or HYBRID 2, for use in the treatment and/or prophylaxis of pruritus, prurigo or neutrophilic dermatoses; wherein the IFN-α14 comprises or consists of an amino acid sequence SEQ ID NO:1; wherein the interferon alpha subtype HYBRID 1 comprises or consists of an amino acid sequence SEQ ID NO:2 and wherein the interferon alpha subtype HYBRID 2 comprises or consists of an amino acid sequence SEQ ID NO:3.

## Patentansprüche

1. Ein Interferon-alpha-Subtyp, wobei der Interferon-alpha-Subtyp IFN-α14, HYBRID 1, HYBRID 2 oder eine Kombination von mindestens zwei von IFN-α14, HYBRID 1 und HYBRID 2 ist, zur Verwendung bei der Behandlung und/oder Prophylaxe von Pruritus, Prurigo oder neutrophilen Dermatosen; wobei das IFN-α14 eine Aminosäuresequenz SEQ ID NO: 1 beinhaltet oder daraus besteht; wobei der Interferon-alpha-Subtyp HYBRID 1 eine Aminosäuresequenz SEQ ID NO: 2 beinhaltet oder daraus besteht; und wobei der Interferon-alpha-Subtyp HYBRID 2 eine Aminosäuresequenz SEQ ID NO: 3 beinhaltet oder daraus besteht.

2. Interferon-alpha-Subtyp gemäß Anspruch 1 zur Verwendung bei der Behandlung und/oder Prophylaxe von Pruritus, Prurigo oder neutrophilen Dermatosen, wobei der Interferon-alpha-Subtyp topisch oder durch sublinguale Verabreichung verabreicht wird.

3. Interferon-alpha-Subtyp gemäß Anspruch 2 zur Verwendung bei der Behandlung und/oder Prophylaxe von Pruritus, Prurigo oder neutrophilen Dermatosen, wobei der Interferon-alpha-Subtyp in einer niedrigen Dosis verabreicht wird; wobei die niedrige Dosis bis zu 5 × 10³ IU/ml oder 5 × 10⁴ IU/ml beträgt.

4. Interferon-alpha-Subtyp gemäß einem der Ansprüche 1 bis 3 zur Verwendung bei der Behandlung eines Zustands, der aus Pruritus oder Prurigo ausgewählt ist.

5. Interferon-alpha-Subtyp gemäß einem der Ansprüche 1 bis 3 zur Verwendung bei der Behandlung von neutrophilen Dermatosen.

6. Interferon-alpha-Subtyp gemäß Anspruch 5 zur Verwendung bei der Behandlung von neutrophilen Dermatosen, wobei die neutrophilen Dermatosen ausgewählt sind aus der Liste, beinhaltend Sweet-Syndrom (SS), neutrophile ekkrine Hidradenitis (NEH), Morbus Beh 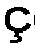 et (BD), Pyoderma gangrenosum (PG), Darm-assoziiertes Dermatitis-Arthritis-Syndrom, rheumatoide neutrophile Dermatitis, Morbus Still des Erwachsenen, primär bullöse, epidermale und vaskulitische NDs.

7. Interferon-alpha-Subtyp gemäß Anspruch 5 zur Verwendung bei der Behandlung von neutrophilen Dermatosen, wobei die neutrophilen Dermatosen ausgewählt sind aus der Liste, beinhaltend Sweet-Syndrom (SS), neutrophile ekkrine Hidradenitis (NEH), Morbus Beh 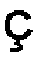 et (BD), Pyoderma gangrenosum (PG), Darm-assoziiertes Dermatitis-Arthritis-Syndrom, rheumatoide neutrophile Dermatitis und Morbus Still des Erwachsenen.

8. Eine Zusammensetzung, die einen Interferon-alpha-Subtyp beinhaltet, wobei der Interferon-alpha-Subtyp IFN-α14, HYBRID 1, HYBRID 2 oder eine Kombination von IFN-α14 und HYBRID 1 oder HYBRID 2 ist, zur Verwendung bei der Behandlung und/oder Prophylaxe von Pruritus, Prurigo oder neutrophilen Dermatosen; wobei das IFN-α14 eine Aminosäuresequenz SEQ ID NO: 1 beinhaltet oder daraus besteht; wobei der Interferon-alpha-Subtyp HYBRID 1 eine Aminosäuresequenz SEQ ID NO: 2 beinhaltet oder daraus besteht und wobei der Interferon-alpha-Subtyp HYBRID 2 eine Aminosäuresequenz SEQ ID NO: 3 beinhaltet oder daraus besteht.

## Revendications

1. Un sous-type de l'interféron alpha, le sous-type de l'interféron alpha étant IFN-α14, HYBRID 1, HYBRID 2 ou une combinaison d'au moins deux sous-types parmi IFN-α14, HYBRID 1, et HYBRID 2 pour son utilisation dans le traitement et/ou la prophylaxie du prurit, du prurigo ou de dermatoses neutrophiliques ; où l'IFN-a14 comprend ou consiste en une séquence d'acides aminés SEQ ID NO : 1 ; où le sous-type de l'interféron alpha HYBRID 1 comprend ou consiste en une séquence d'acides aminés SEQ ID NO : 2 ; et où le sous-type de l'interféron alpha HYBRID 2 comprend ou consiste en une séquence d'acides aminés SEQ ID NO : 3.

2. Le sous-type de l'interféron alpha de la revendication 1 pour son utilisation dans le traitement et/ou la prophylaxie du prurit, du prurigo ou de dermatoses neutrophiliques, le sous-type de l'interféron alpha étant administré par voie topique ou étant à administration sublinguale.

3. Le sous-type de l'interféron alpha de la revendication 2 pour son utilisation dans le traitement ou la prophylaxie du prurit, du prurigo ou de dermatoses neutrophiliques, le sous-type de l'interféron alpha étant administré à raison d'une faible dose ; où la faible dose va jusqu'à 5 × 10³ UI/ml ou 5 × 10⁴ UI/ml.

4. Le sous-type de l'interféron alpha de n'importe lesquelles des revendications 1 à 3 pour son utilisation dans le traitement d'un état sélectionné parmi le prurit, ou le prurigo.

5. Le sous-type de l'interféron alpha de n'importe lesquelles des revendications 1 à 3 pour son utilisation dans le traitement de dermatoses neutrophiliques.

6. Le sous-type de l'interféron alpha de la revendication 5 pour son utilisation dans le traitement de dermatoses neutrophiliques, les dermatoses neutrophiliques étant sélectionnées dans la liste comprenant le syndrome de Sweet (SS), l'hidradénite eccrine neutrophilique (HEN), la maladie de Behçet (BD), le pyoderma gangrenosum (PG), le syndrome dermatose-arthrite associé à l'intestin, la dermatose neutrophilique rhumatoïde, la maladie de Still de l'adulte, des DN essentiellement bulleuses, épidermiques, et vasculitiques.

7. Le sous-type de l'interféron alpha de la revendication 5 pour son utilisation dans le traitement de dermatoses neutrophiliques, les dermatoses neutrophiliques étant sélectionnées dans la liste comprenant le syndrome de Sweet (SS), l'hidradénite eccrine neutrophilique (NEH), la maladie de Behçet (BD), le pyoderma gangrenosum (PG), le syndrome dermatose-arthrite associé à l'intestin, la dermatose neutrophilique rhumatoïde, et la maladie de Still de l'adulte.

8. Une composition comprenant un sous-type de l'interféron alpha, le sous-type de l'interféron alpha étant IFN-α14, HYBRID 1, HYBRID 2 ou une combinaison d'IFN-a14 et d'HYBRID 1 ou HYBRID 2, pour son utilisation dans le traitement et/ou la prophylaxie du prurit, du prurigo ou de dermatoses neutrophiliques ; où l'IFN-a14 comprend ou consiste en une séquence d'acides aminés SEQ ID NO : 1 ; où le sous-type de l'interféron alpha HYBRID 1 comprend ou consiste en une séquence d'acides aminés SEQ ID NO : 2 ; et où le sous-type de l'interféron alpha HYBRID 2 comprend ou consiste en une séquence d'acides aminés SEQ ID NO : 3.
